Europäisches Patentamt

European Patent Office

Office européen des brevets

(11) Numéro de publication: **0 177 409**
**A1**

# (12) DEMANDE DE BREVET EUROPEEN

(21) Numéro de dépôt: **85401865.2**

(22) Date de dépôt: **25.09.85**

(51) Int. Cl.⁴: **A 61 K 35/50**
**A 61 K 7/48**

(30) Priorité: **26.09.84 FR 8414802**

(43) Date de publication de la demande:
**09.04.86 Bulletin 86/15**

(84) Etats contractants désignés:
**AT BE CH DE FR GB IT LI LU NL SE**

(71) Demandeur: **Laumond, Gérard Dr.**
**3, Rue de la Fontaine**
**F-64200 Biarritz(FR)**

(72) Inventeur: **Laumond, Gérard**
**3, Rue La Fontaine**
**F-64200 Biarritz(FR)**

(72) Inventeur: **Bailly, Alain**
**15, Rue des Bouvreuils**
**F-91540 Mennecy(FR)**

(74) Mandataire: **Gillard, Marie-Louise et al,**
**Cabinet Beau de Loménie 55, Rue d'Amsterdam**
**F-75008 Paris(FR)**

(54) **Composition, utile notamment en cosmétologie.**

(57) La présente invention concerne une composition utile en cosmétologie, caractérisée en ce qu'elle est obtenue par ultrafiltration stérilisante à partir d'une solution contenant:

- de 40 à 60 % en volume de liquide amniotique embryonnaire prélevé sur des mammifères,

- de 40 à 60 % en volume d'un sérum, choisi parmi les sérums de bovidés, d'équidés ou de leur mélange, prélevé sur des animaux exempts de vaccination depuis plus de trois mois.

Ladite composition stimule notamment le métabolisme des fibroblastes de la peau.

EP 0 177 409 A1

Croydon Printing Company Ltd.

Composition, utile notamment en cosmétologie.

La présente invention a pour objet une composition, utile notamment en cosmétologie et plus particulièrement en dermatologie.

Il s'agit d'une solution à usage externe. Ses applications sont multiples. On soulignera son pouvoir cicatrisant et son pouvoir anti-ride.

Ladite composition selon l'invention est à base d'un mélange de liquide amniotique et de sérum, d'origine animale. Elle se caractérise par une grande propreté bactériologique, due notamment à son procédé de fabrication et une réelle efficacité, due au choix de ses constituants et au mode de prélèvement desdits constituants.

Des compositions de ce type ont déjà été décrites, notamment dans la demande FR 2 160 285. Il est question dans cette demande de composés de sérum, constitués d'un lyophilisat de sérum embryonnaire de bovin ou de brebis, de sérum de veau, de liquide amniotique et de placenta. La propreté bactériologique de tels composés ne peut être garantie. Leur mode de stérilisation -lyophilisation- ne répond notamment plus aux normes officielles françaises de préparation de cosmétiques. Il a d'autre part été démontré que la lyophilisation de ce type de composés entamait leur efficacité.

Selon la présente invention, il a été mis au point une composition, répondant à des normes de qualité jamais décrites auparavant, afin de garantir son efficacité et innocuité et de se soumettre à des réglementations strictes en matière de cosmétologie.

La composition selon l'invention est caractérisée en ce qu'elle est obtenue par ultrafiltration stérilisante à partir d'une solution contenant :

- de 40 à 60 % en volume de liquide amniotique embryonnaire prélevé sur des mammifères, et

- de 40 à 60 % en volume d'un sérum, choisi parmi les sérums de bovidés, d'équidés ou de leurs mélanges, prélevés sur des animaux exempts de vaccination depuis plus de trois mois.

Le liquide amniotique utilisé est prélevé sur n'importe quel mammifère, contrôlé sanitairement, le prélèvement ayant lieu au cours des premiers mois de gestation. Il s'agit de répondre tout d'abord à un critère de pureté.

A partir d'un certain développement du foetus, le liquide amniotique est pollué par celui-ci. On a d'autre part noté que l'efficacité du produit final était liée à la précocité dudit prélèvement.

Pour des raisons pratiques, on prélève généralement ledit liquide amniotique embryonnaire sur des bovidés. Le prélèvement sur ces animaux, contrôlés sanitairement, est effectué avant le quatrième mois de gestation.

Le sérum intervenant dans la composition selon l'invention est prélevé sur des animaux -bovidés ou équidés- contrôlés sanitairement et exempts de vaccination depuis plus de trois mois. On évite ainsi la présence d'anticorps dans la solution selon l'invention.

La composition selon l'invention se caractérise également par son procédé de stérilisation : ultrafiltration. Il s'agit d'une filtration sous pression, faisant intervenir un ultrafiltre. Le filtre utilisé présente des pores d'environ 0,20 microns de diamètre, ce afin d'arrêter les microbes contenus dans la solution.

On utilisera par exemple des filtres Millipores, ayant des pores de 0,22 microns, sous une pression de 2 à 3 bars.

Ce type de stérilisation, contrairement à la lyophilisation assure à la composition selon l'invention la propreté bactériologique requise sans diminuer son efficacité.

Ladite composition peut contenir en outre un ou des parfums et un ou des agents stabilisateurs.

Le parfum, que l'on ajoute avantageusement, notamment pour la préparation de cosmétiques, est destiné à masquer l'odeur désagréable du mélange -liquide amniotique, sérum- et à aromatiser la solution. On utilise tout parfum, soluble dans le mélange, à la seule condition qu'il ne contienne pas d'alcool. La présence d'alcool impliquerait notamment une certaine destruction des cellules embryonnaires du liquide amniotique. Dans la gamme de parfums, autorisés par telle ou telle législation, on choisira suivant les goûts. On utilisera par exemple une base aromatique végétale. L'agent stabilisateur permet à la solution selon l'invention de rester claire. On utilisera un agent stabilisateur classique et par exemple le mélange de 0,6 parties pour 1000 de para-hydroxy benzoate de méthyle sodique et de 0,4 parties pour 1000 de para-hydroxy benzoate de propyle sodique.

Eventuellement, on ajoutera également à la composition selon l'invention un agent conservateur. L'emploi d'un tel agent n'est pas obligatoire, car comme indiqué ci-dessus, au cours de son procédé de fabrication, la solution selon l'invention est rendue parfaitement stérile. Par précaution supplémentaire vis-à-vis de certains utilisateurs négligents, on peut toutefois ajouter un tel agent. Il sera choisi parmi les conservateurs classiques, utilisables en cosmétologie. La composition selon l'invention pourra notamment contenir du KATHON CG. Il s'agit d'un conservateur récent qui associe une grande activité à faible dose à un pouvoir irritant pratiquement nul. Ce produit commercialisé est un dérivé thiazinique. Il titre 1,5 % de matière active, composée de deux produits : le 5-chloro 2-méthyl 4-isothiazoline 3-one (75 %) et le 2-méthyl 4-isothiazoline 3-one (25 %), % exprimés en volume. Il est parfaitement soluble dans la composition selon l'invention. On peut l'employer à raison d'environ 1 g/l de solution.

La composition selon l'invention résulte du simple mélange de ces constituants. On effectue tout d'abord les prélèvements de liquide amniotique et de sérum, dans les conditions indiquées ci-dessus.

On ajoute éventuellement au mélange de ces deux constituants de base le parfum et l'agent stabilisateur, éventuellement encore l'agent conservateur.

On indiquera ici, et il s'agit d'un autre objet de la présente invention, que le mélange desdits constituants -liquide amniotique, sérum- peut également servir de base à la préparation de divers produits cosmétiques contenant différents tissus embryonnaires non lyophilisés tels que : placenta, mesenchyine, thymus, foie...

Le mélange des différents ingrédients ainsi réalisé -liquide amniotique, sérum et éventuellement autres produits précisés ci-dessus- est stérilisé par ultrafiltration.

La composition selon l'invention ainsi obtenue, parfaitement stérile, peut être conservée par congélation ou être conditionnée de suite pour sa commercialisation. On effectue par exemple sa mise en ampoules sous flux laminaire.

Un tel conditionnement associé à l'ultrafiltration stérilisante permet de fabriquer et commercialiser la composition selon l'invention sans adjonction de conservateur, ni de stabilisateur. Le produit ainsi obtenu peut être conservé très longtemps (plus d'un an) par congélation.

La composition selon l'invention exerce sur la peau un effet bénéfique. Quel que soit le sérum utilisé, en association avec le liquide amniotique, elle présente un pouvoir réparateur, cicatrisant incontestable.

Par ces éléments constitutifs, elle nourrit la peau par apport notamment de matières grasses, elle l'hydrate et en accélère la reconstitution. Son action sur le métabolisme des fibroblastes qui met en jeu les réactions de synthèse du collagène et de l'élastine de la peau a été mise en évidence.

La composition selon l'invention intervient donc favorablement dans l'atténuation des rides, dans la prévention des vergétures, le traitement des brûlures superficielles par le feu, le soleil ou d'autres rayons et dans la disparition des marques de blessures ou de rougeurs, quelqu'en soit l'origine. Dans le traitement des brûlures sans plaies, elle a certaines propriétés analgésiques.

Chacun des principaux éléments de la composition selon l'invention -liquide amniotique, sérum- possède lesdites propriétés mais à un degré moindre. Leur association met sans nul doute un effet de synergie en jeu.

D'autre part, on a remarqué que lorsque le sérum utilisé dans le mélange était du sérum d'équidés, la composition selon l'invention se distinguait avant tout par son pouvoir anti-ride. Elle se révèle alors un véritable tenseur de la peau.

On notera enfin que les compositions selon l'invention ne présentent aucun caractère toxique.

On précise ci-après les résultats obtenus avec deux compositions préférées selon l'invention. Ces exemples sont donnés à titre d'illustration et ne sont pas limitatifs.

COMPOSITION A

Elle est réalisée à partir d'un mélange de 55 % en volume de liquide amniotique de bovidés et de 45 % en volume de sérum de bovidés.

Ladite composition A a été dénommée formule normale. Elle possède entre autre un très bon pouvoir cicatrisant. On a testé celui-ci sur des brûlures et des contusions. La composition A est passée, à raison de 2 fois par jour, sur les lésions. On a constaté au bout de 8 jours une cicatrisation complète avec disparition de la cicatrice.

COMPOSITION B

Elle est réalisée à partir d'un mélange de 50 % en volume de liquide amniotique de bovidés et de 50 % en volume de sérum d'équidés.

Ladite composition B est dénommée formule forte. Elle a les mêmes propriétés que la formule normale mais elle se montre un bien meilleur tenseur de la peau. Ainsi, une nette atténuation des rides peut être constatée après application de la composition B, régulièrement une fois par jour. Vingt minutes après une première application de ladite composition, on "sent" la peau se tendre.

Suivant l'effet recherché, on appliquera la composition A ou B. Elles peuvent être utilisées en combinaison.

Par exemple, en cas de forts coups de soleil, on utilisera tout d'abord la formule normale et seulement après plusieurs applications de celle-ci, on emploiera la formule forte.

On a mis en évidence par le test présenté ci-après -protocole et résultats- l'effet stimulant de ladite composition B sur la prolifération des fibroblastes.

On utilise un ensemencement de fibroblastes de la lignée L 929 en boîtes de Petri de 35 mm à raison de $7 \times 10^4$ cellules par boîtes. La culture est réalisée en milieu MEM contenant des antibiotiques (Pénicilline-Streptomycine) et 2,5 % de sérum de veau, à 37°C dans une atmosphère régulée en $CO_2$ (5 %).

Après 24 heures de culture, on remplace ledit milieu de culture par, d'une part, un milieu neuf contenant différentes concentrations du produit à étudier (2,5 %, 1 %, 0,5 %, 0,1 % de la compo-

0177409

sition B) et d'autre part par un milieu neuf contenant 2,5 et 5 % de sérum de veau (milieux témoins).

Après trois jours de traitement, on étudie la croissance par numération cellulaire après décollement par la trypsine. Chaque variable est effectuée en triple et deux numérations sont réalisées par boîte de Petri. Les résultats sont formulés par la moyenne des 6 valeurs obtenues et indiquées dans le tableau ci-dessous :

|  | NOMBRE DE CELLULES PAR BOITE | % RELATIF |
|---|---|---|
| Témoin 5 % | 1 840 000 | |
| Témoin 2,5 % | 1 020 000 | |
| Composition B  2,5 % | 1 865 000 | 183 % |
| Composition B  1 % | 1 430 000 | 140 % |
| Composition B  0,5 % | 1 360 000 | 133 % |
| Composition B  0,1 % | 1 180 000 | 116 % |

( % relatif / témoin 2,5 % sérum de veau foetal)

Ces résultats montrent qu'à une concentration de 2,5 % la composition B stimule très nettement la prolifération des fibroblastes ; cette stimulation de la prolifération cellulaire est déjà mise en évidence à des concentrations de 0,5, 1 %.

Lesdits résultats démontrent l'action particulièrement bénéfiques des compositions selon l'invention sur les cellules de la peau.

Ladite action ne se limite pas à un apport d'éléments mais se situe également à un second degré. Les compositions selon l'invention agissent directement sur le métabolisme des cellules de la peau.

REVENDICATIONS

1.        Composition, utile notamment en cosmétologie, caractérisée
en ce qu'elle est obtenue par ultrafiltration stérilisante à partir
d'une solution contenant :
            - de 40 à 60 % en volume de liquide amniotique embryonnaire
prélevé sur des mammifères,
            - de 40 à 60 % en volume d'un sérum, choisi parmi les sérums
de bovidés, d'équidés ou de leur mélange, prélevés sur des animaux
exempts de vaccination depuis plus de trois mois.
2.        Composition selon la revendication 1, caractérisée en ce
que le liquide amniotique embryonnaire est prélevé sur des bovidés.
3.        Composition selon l'une des revendications 1 ou 2, caractérisée en ce qu'elle contient :
            - 55 % en volume de liquide amniotique embryonnaire prélevé
sur des bovidés,
            - 45 % en volume de sérum de bovidés, prélevé sur des animaux exempts de vaccination depuis plus de trois mois.
4.        Composition selon l'une des revendications 1 ou 2, caractérisée en ce qu'elle contient :
            - 50 % en volume de liquide amniotique embryonnaire prélevé
sur des bovidés,
            - 50 % en volume de sérum d'équidés, prélevé sur des animaux
exempts de vaccination depuis plus de trois mois.
5.        Composition selon l'une quelconque des revendications 1 à 4,
caractérisée en ce qu'elle contient en outre un agent stabilisateur,
un parfum et éventuellement un agent conservateur.
6.        Composition selon l'une quelconque des revendications 1 à 5,
caractérisée en ce qu'elle contient en outre un ou des tissus embryonnaires non lyophilisés tel que placenta, mesenchyine, thymus, foie...

REVENDICATIONS (pour l'état contractant : AT)

1.      Procédé pour la préparation d'une composition utile notamment en cosmétologie caractérisé  en ce qu'il consiste à réaliser un mélange contenant :

        - de 40 à 60 % en volume de liquide amniotique embryonnaire prélevé sur des mammifères,

        - de 40 à 60 % en volume d'un sérum, choisi parmi les sérums de bovidés, d'équidés ou de leur mélange, prélevés sur des animaux exempts de vaccination depuis plus de trois mois et à stériliser ledit mélange obtenu par ultrafiltration.

2.      Procédé selon la revendication 1, caractérisé en ce que le liquide amniotique embryonnaire est prélevé sur des bovidés.

3.      Procédé selon l'une des revendications 1 ou 2, caractérisé en ce que le mélange contient :

        - 55 % en volume de liquide amniotique embryonnaire prélevé sur des bovidés,

        - 45 % en volume de sérum de bovidés, prélevé sur des animaux exempts de vaccination depuis plus de trois mois.

4.      Procédé selon l'une des revendications 1 ou 2, caractérisé en ce que le mélange contient :

        - 50 % en volume de liquide amniotique embryonnaire prélevé sur des bovidés,

        - 50 % en volume de sérum d'équidés prélevé sur des animaux exempts de vaccination depuis plus de trois mois.

5.      Procédé selon l'une quelconque des revendications 1 à 4, caractérisé en ce que le mélange contient en outre un agent stabilisateur, un parfum et éventuellement un agent conservateur.

6.      Procédé selon l'une quelconque des revendications 1 à 5, caractérisé en ce que le mélange contient en outre un ou des tissus embryonnaires non lyophilisés tel que placenta, mesenchyine, thymus, foie...

## Office européen
## des brevets

# RAPPORT DE RECHERCHE EUROPEENNE

## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int Cl 4) |
|---|---|---|---|
| D,X | FR-A-2 160 285 (A.N. METZINGER) <br> * Page 3, lignes 1-14; revendications 1-3 * <br><br> ----- | 1-6 | A 61 K 35/50 <br> A 61 K 7/48 |
| | | | **DOMAINES TECHNIQUES RECHERCHES (Int Cl 4)** <br><br> A 61 K |

Le présent rapport de recherche a été établi pour toutes les revendications

| Lieu de la recherche <br> LA HAYE | Date d'achèvement de la recherche <br> 06-01-1986 | Examinateur <br> BRINKMANN C. |
|---|---|---|

OEB Form 1503 03 82

CATEGORIE DES DOCUMENTS CITES

X : particulièrement pertinent à lui seul
Y : particulièrement pertinent en combinaison avec un
autre document de la même catégorie
A : arrière-plan technologique
O : divulgation non-écrite
P : document intercalaire

T : théorie ou principe à la base de l'invention
E : document de brevet antérieur, mais publié à la
date de dépôt ou après cette date
D : cité dans la demande
L : cité pour d'autres raisons

& : membre de la même famille, document correspondant